# EUROPÄISCHE PATENTANMELDUNG

(11) **EP 3 235 453 A1**
(43) Veröffentlichungstag der Anmeldung: **25.10.2017**
(21) Anmeldenummer: 16166190.5
(22) Anmeldetag: 20.04.2016
(51) Int. Cl.: A61B 17/17

(54) **KNOCHENBEARBEITUNGS-FÜHRUNGSSCHIENE, ANORDNUNG UMFASSEND ZUMINDEST EINE ERSTE KNOCHENBEARBEITUNGS-FÜHRUNGSSCHIENE UND VERFAHREN ZUM BEREITSTELLEN VON SCHIENENABSCHNITTEN EINER MEHRTEILIGEN KNOCHENBEARBEITUNGS-FÜHRUNGSSCHIENE**

(71) Anmelder: Universität Basel, 4003 Basel (CH)
(72) Erfinder: Zillig, Timo, 4310 Rheinfelden, AG (CH); Stübinger, Stefan, 4102 Binningen, BL (CH)
(74) Vertreter: Kleine, Hubertus

(57) **Zusammenfassung**

Eine Knochenbearbeitungs-Führungsschiene zur Halterung eines Operationsinstruments an einem Knochen und/oder einer Osteosynthese, wobei die Knochenbearbeitungs-Führungsschiene eine Laufbahn zur Führung des Operationsinstruments entlang einer vorbestimmten Verlaufsstrecke aufweist, sowie eine Anordnung umfassend zumindest eine erste Knochenbearbeitungs-Führungsschiene und ein Verfahren zum Bereitstellen Schienenabschnitten einer mehrteiligen Knochenbearbeitungs-Führungsschiene, insbesondere einer Knochenbearbeitungs-Führungsschiene nach einem der vorhergehenden Schritte, vor deren Einführung in einen menschlichen und/oder tierischen Körper.

## Beschreibung

Die vorliegende Erfindung betrifft eine Knochenbearbeitungs-Führungsschiene, sowie eine Anordnung umfassend zumindest eine erste Knochenbearbeitungs-Führungsschiene und ein Verfahren zum Bereitstellen von Schienenabschnitten einer mehrteiligen Knochenbearbeitungs-Führungsschiene.

Als Osteotomie wird ein chirurgisches Verfahren bezeichnet, bei welchem Knochen gezielt durchtrennt werden. Es wird angewandt um beispielsweise ein fehlerhaftes Verwachsen von Knochen zu verhindern. Eine entsprechende Bearbeitung des Knochens erfolgt mittels eines Operationsinstruments, beispielsweise mittels einer Säge oder eines Lasers. Derartige Operationsinstrumente werden freihandgeführt.

Ausgehend von dieser Vorgehensweise ist es nunmehr Aufgabe der vorliegenden Erfindung dem Chirurgen eine Erleichterung der Führung des Operationsinstruments zur Verfügung zu stellen.

Die Erfindung löst diese Aufgabe durch eine Knochenbearbeitungs-Führungsschiene mit den Merkmalen des Anspruchs 1, sowie durch eine Anordnung mit den Merkmalen des Anspruchs 8.

Zudem wird ein Fertigungsverfahren für eine entsprechende Knochenbearbeitungs-Führungsschiene im Rahmen der vorliegenden Erfindung beschrieben.

Eine erfindungsgemäße Knochenbearbeitungs-Führungsschiene zur Halterung eines Operationsinstruments an einem Knochen weist eine Laufbahn zur Führung des Operationsinstruments entlang einer vorbestimmten Verlaufsstrecke auf. Ein Knochen im Rahmen der vorliegenden Erfindung kann jede erdenkliche Form eines Knochens sein, so z.B. ein Röhrenknochen, ein platter Knochen, ein kurzer Knochen, ein Sesamknochen oder dergleichen. Auch an einer sogenannten Osteosynthese, also an einem künstlicher Knochen oder einem Knochensegment zur Verbindung mit weiteren Knochen kann die Knochenbearbeitungs-Führungsschiene angebracht werden.

Eine Knochenbearbeitungs-Führungsschiene kann zur Halterung eines Osteotomie-Operationsinstruments also einem knochendurchtrennenden Operationsinstrument, wie z.B. einem Laser, Bohrer oder einer Säge, dienen. Auch komplexere Operationsinstrumente wie z.B. ein Endoskop können in der Halterung aufgenommen werden.

Die Knochenbearbeitungs-Führungsschiene kann jedoch auch der Halterung und Führung von anderen knochenbearbeitenden Operationsinstrumenten dienen, so z.B. von Bohrern, Fräsen oder Schleifgeräten.

Eine erfindungsgemäße Knochenbearbeitungs-Führungsschiene kann auch plattenförmig ausgebildet sein und einen Spalt mit einem Verlauf über die vorbestimmte Verlaufsstrecke aufweisen. In diesem Fall ist die Laufbahn nicht an einer Außenseite eines Schienenstrangs ausgebildet sondern in dem Spalt.

Eine Laufbahn im Rahmen der vorliegenden Erfindung ist eine Bahn, auf welche ein gleitender, walzender und/oder rollender Körper aufliegt. Die Laufbahn kann durch eine Schiene oder durch eine auf der Schiene aufgebrachte Beschichtung ausgebildet sein.

Die Verlaufsstrecke kann beispielsweise einen unregelmäßigen gewundenen Verlauf aufweisen. Es kann sich bei der Verlaufsstrecke allerdings auch um eine Schnittgerade handeln. Die Verlaufsstrecke wird durch die Länge der Laufbahn definiert. Sie kann allerdings auch deutlich kürzer sein als die Knochenbearbeitungs-Führungsschiene.

Führungsschienen zur Führung von Operationsinstrumenten, insbesondere mit aktiven Elementen, sind in der Osteotomie bislang nicht bekannt.

Vorteilhafte Ausgestaltungen der Erfindung sind Gegenstand der Unteransprüche.

Es ist von Vorteil wenn die Knochenbearbeitungs-Führungsschiene aus zumindest zwei oder mehr Schienenabschnitten gebildet ist, welche gemeinsam die Laufbahn ausbilden. Durch die Mehrteiligkeit der Führungsschiene ist diese minimalinvasiv durch die Haut einführbar und kann im Körper zusammengesetzt werden.

Es ist von Vorteil, wenn die Führungsschiene aus zumindest einem ersten Schienenabschnitt und einem zweiten Schienenabschnitt aufweist wobei der erste Schienenabschnitt einen ersten Laufbahnabschnitt und der zweite Schienenabschnitt einen zweiten Laufbahnabschnitt aufweist, wobei der ersten Laufbahnabschnitt eine stärkere Biegung aufweist als der zweite Laufbahnabschnitt.

Es ist zudem von Vorteil, wenn die Führungsschiene, insbesondere jeder der Schienenabschnitte, eine Kontaktfläche zur Auflage auf einem Knochen aufweist.

Die Laufbahn der Führungsschiene kann vorteilhaft eine magnetische Beschichtung aufweisen. Sensoren, welche beispielsweise in der Halterung integriert sind, können zudem vorteilhaft die Position des Operationsinstruments bestimmen.

Es ist von Vorteil, wenn zumindest mehr als 50% aller Schienenabschnitte unterschiedlich zueinander dimensioniert gefertigt sind. Dadurch können gerade Teilstrecken und Biegungen vorgesehen sein.

Die Schienenabschnitte definieren vorteilhaft im zusammengesetzten Zustand einer Knochenbearbeitungs-Führungsschiene ein Kantenmodell mit einem größeren Volumen als jeder einzelne Schienenabschnitt, vorzugsweise ein dreifach größeres Kantenmodell, insbesondere ein 8-fach größeres Kantenmodell.

Die Knochenbearbeitungs-Führungsschiene kann zudem Anschlussstellen, vorzugsweise für Steckverbindungen, zur Fixierung eines Knochensynthese und/oder eines Implantats an der Knochenbearbeitungs-Führungsschiene aufweisen. Dadurch hat die Knochenbearbeitungs-Führungsschiene auch nach dem durchgeführten chirurgischen Eingriff eine Verankerungsfunktion für im Körper verbleibende Elemente. Die Knochenbearbeitungs-Führungsschiene selbst kann dabei aus biologisch-abbaubarem Material gebildet sein.

Eine erfindungsgemäße Anordnung umfasst zumindest eine erste Knochenbearbeitungs-Führungsschiene, insbesondere eine erfindungsgemäße erste Knochenbearbeitungs-Führungsschiene und eine Halterung für ein Operationsinstrument zur Bearbeitung eines Knochens und/oder ein Operationsinstrument zur Bearbeitung eines Knochens mit einer Halterung, wobei die Halterung eine wälz- und/oder gleitgelagert an der ersten Knochenbearbeitungs-Führungsschiene angeordnet ist.

Die Anordnung kann somit entweder nur eine Halterung oder ein komplettes Operationsinstrument einschließlich der Halterung umfassen, welche in diesem Fall dem Operationsinstrument zugeordnet ist.

Vorteilhafte Ausgestaltungen der Erfindung sind Gegenstand der Unteransprüche.

Die Anordnung kann vorteilhaft eine zweite Knochenbearbeitungs-Führungsschiene, insbesondere eine zweite Knochenbearbeitungs-Führungsschiene nach einem der vorhergehenden Ansprüche, aufweist, wobei jede der beiden Führungsschienen jeweils eine Laufbahn aufweist, wobei beide Laufbahnen Teil eines Führungskanals sind, in welchem das Operationsinstrument mittels der Halterung gleit- und/oder wälzgelagert angeordnet ist.

Die Halterung kann vorteilhaft eine Kugelform aufweisen, welche auf den Laufbahnen aufliegt oder ein zylindrisches Wälzlager aufweisen, welches auf den Laufbahnen aufliegt.

Die Anordnung kann einen Kabelführungskanal aufweisen, zur Abdeckung eines Kabels des Operationsinstruments. Bei Installation der Anordnung unter der Haut eines Patienten kann das Operationsinstrument besser bewegt werden. Unangenehme Reibungen durch ein Stromversorgungskabel im Körper werden dabei vermieden.

Die erfindungsgemäße Knochenbearbeitungs-Führungsschiene und die erfindungsgemäße Anordnung oder deren vorbeschriebene vorteilhafte Ausgestaltungsvarianten weisen eine Vielzahl von weiteren Vorteile auf.

Die Schnittgeometrie für einen Eingriff am Knochen ist vor dem Eingriff frei wählbar und kann entsprechend bei der Fertigung der Knochenbearbeitungs-Führungsschiene berücksichtigt werden.

Der Schnitt-, Bohr- oder Schliffwinkel ist vor der Operation frei vom Operateur wählbar und kann bei der geometrischen Ausgestaltung der Knochenbearbeitungs-Führungsschiene berücksichtigt werden. Dieser Winkel kann sich während der Operation auch mehrfach ändern und entsprechende Änderungen können bei der Gestaltung berücksichtigt werden.

Es ist zudem möglich während der Operation das Operationsinstrument auszuwechseln.

Eine Fixierung des Patienten, wie dies im Fall einer roboterunterstützten Operation erfolgen muss, kann bei Einsatz einer erfindungsgemäßen Knochenbearbeitungs-Führungsschiene und/oder einer erfindungsgemäßen Anordnung entfallen.

Die Knochenbearbeitungs-Führungsschiene kann einen oder mehrere Sensoren und/oder Aktuatoren aufweisen zur Überwachung der Operationsbedingungen, z.B. der Körpertemperatur an der Stelle des chirurgischen Eingriffs.

Durch den Einsatz der Führungsschiene kann das Operationsinstrument sowohl von Hand oder mittels eines Roboters geführt werden.

Wie bereits beschrieben stellt eine mehrteilige Ausgestaltung der Knochenbearbeitungs-Führungsschiene aus Gründen einer minimalinvasiven Operationsstrategie einen besonderen Vorteil dar.

Ebenfalls erfindungsgemäß ist daher ein Verfahren zum Bereitstellen von Schienenabschnitten einer mehrteiligen Knochenbearbeitungs-Führungsschiene, insbesondere einer Knochenbearbeitungs-Führungsschiene nach einem der vorhergehenden Schritte, vor deren Einführung in einen menschlichen und/oder tierischen Körper. Dabei ist das Verfahren gekennzeichnet durch die folgenden Schritte:
A) Erfassen von Daten eines Patienten, für welchen die Knochenbearbeitungs-Führungsschiene bestimmt ist;
B) Erstellen eines Modells anhand der erfassten Daten;
C) Erstellen von Fertigungsvorgaben für zumindest zwei oder mehr zu einer Knochenbearbeitungs-Führungsschiene zusammensetzbaren Schienenabschnitten auf Grundlage des erstellten Modells wobei die Fertigungsvorgaben umfassen:
   C1) eine Dimensionierung der Schienenabschnitte und
D) Fertigung der Schienenabschnitte auf Basis der Fertigungsvorgaben; wobei die Schienenabschnitte miteinander zu einer Knochenbearbeitungs-Führungsschiene montierbar sind.

Ausgehend von dem vorbeschriebenen Verfahren kann eine patientenspezifische Erstellung von Schienenabschnitten einer Knochenbearbeitungs-Führungsschiene erfolgen. Da jeder Patient abhängig von Körpergrößen und anderen Merkmalen eine andere Knochengeometrie besitzt kann die Führungsschiene bzw. deren Einzelteile vor der Operation individuell für den Patienten hergestellt und im Anschluss in den Patienten eingesetzt und dort zusammengesetzt und am Knochen festgelegt werden.

Vorteilhafte Ausgestaltungen des Fertigungsverfahrens sind Gegenstand der Unteransprüche.

Vorteilhaft kann das Erstellen von Fertigungsvorgaben für zumindest zwei oder mehr zu einer Knochenbearbeitungs-Führungsschiene zusammensetzbaren Schienenabschnitte auf Grundlage des erstellten Modells erfolgen, wobei die Fertigungsvorgaben umfassen:
C2) eine Materialauswahl bezüglich eines oder mehrerer Materialen für einen jeweiligen Schienenabschnitt;
C3) eine Auswahl einer oder mehrerer Verbindungarten zwischen den jeweiligen Schienenabschnitten.

Es kann beispielsweise gefordert sein, dass Teile bzw. Schienenabschnitte der Knochenbearbeitungs-Führungsschiene eine höhere Flexibilität aufweisen als andere Teile der Knochenbearbeitungs-Führungsschiene, beispielsweise um Krümmungen noch im Körper besser an die Gegebenheiten des Knochens anzupassen. Dies kann durch eine entsprechende Materialauswahl während der Fertigung berücksichtigt werden.

Es kann ebenfalls gefordert sein, dass einzelne Schienenabschnitte leicht zerlegbar sind, um die Knochenbearbeitungs-Führungsschiene aus dem Körper nach dem erfolgten chirurgischen Eingriff zu entfernen, während Schienenabschnitte an anderen Stelle einen besonders festen Halt benötigen. Dies kann durch Auswahl verschiedener Verbindungsarten zwischen den Schienenabschnitten, z.B. durch starre Verbindungen oder Gelenkverbindungen, berücksichtigt werden.

Das Erstellen des Modells kann als Erstellen eines dreidimensionalen Computer-Modells, vorzugsweise nach der Finite Elemente Methode, erfolgen. Diese Methode ist an sich bekannt und kann nunmehr zur Modellierung der komplizierten Struktur von Schienenabschnitte eingesetzt werden. Alternativ kann auch ein dreidimensionales Modell des betreffenden Knochens vorab in einem 3-D Druckverfahren gedruckt werden und eine Anpassung der Schienenabschnitte anhand dieses dreidimensionalen Modells erfolgen.

Zur Erstellung des Modells kann vorteilhaft und patientenindividuell durch das Erfassen von Daten durch ein bildgebendes Verfahren zum Zwecke des Erstellens eines dreidimensionalen Modells erfolgen.

Insgesamt wird durch das vorbeschriebene Verfahren eine hohe Exaktheit bei der voroperativen Planung eines Eingriffs am Knochen erreicht und es kann eine Führungsschiene hergestellt werden, welche perfekt auf den Operationsbereich des Patienten abgestimmt ist.

Die Knochenbearbeitungs-Führungsschiene kann teilweise oder vollständig nach erfolgtem Eingriff auch als Verankerungsvorrichtung zur Verankerung, z.B. von Implantaten, Osteosynthesen und/oder Verbindungsmitteln zur Verbindung derselben mit einem Knochen, an der Operationsstelle verwendet werden. Teilweise bedeutet dabei, dass nur Teile der Knochenbearbeitungs-Führungsschiene am Knochen fixiert zurückbleiben und zur Verankerung weiterer Elemente dienen können.

Die vorliegende Erfindung wird anhand mehrerer Ausführungsbeispiele und unter Zuhilfenahme der beiliegenden Figuren näher erläutert. Es zeigt:
- Fig. 1: schematische Darstellung einer erfindungsgemäßen Anordnung umfassend ein Operationsinstrument und eine erfindungsgemäße Knochenbearbeitungs-Führungsschiene zur Führung desselben entlang einer vorgegebenen Strecke entlang eines Knochensegments;
- Fig. 2: Draufsicht der erfindungsgemäßen Anordnung der Fig. 1;
- Fig. 3: perspektivische Schnittansicht der Anordnung der Fig. 1 und 2;
- Fig. 4: Perspektivansicht der Anordnung der Fig. 1-3 auf einem Knochen;
- Fig. 5: Teilausschnitt der Knochenbearbeitungsführungsschiene der Fig. 1-4;
- Fig. 6: Ablaufdiagramm für ein Herstellungsverfahren einer erfindungsgemäßen Knochenbearbeitungs-Führungsschiene;
- Fig. 7: eine Darstellung einer zweiten Ausführungsvariante einer erfindungsgemäßen Anordnung und einer erfindungsgemäßen Knochenbearbeitungs-Führungsschiene;
- Fig. 8: eine Detailansicht der Anordnung der Fig. 7.

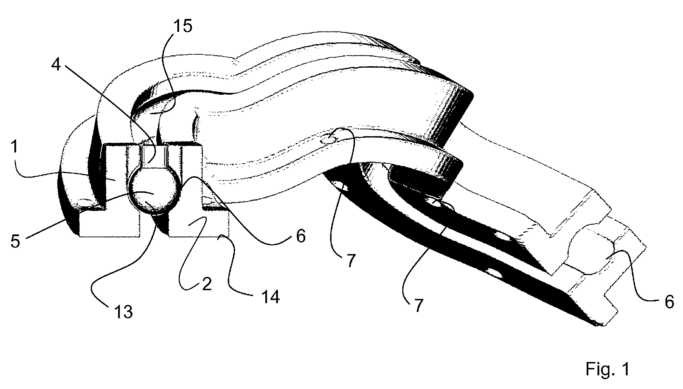

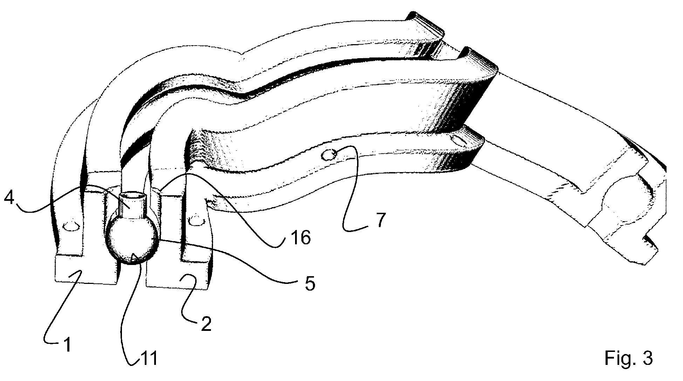

Fig. 1-4 zeigt eine schematische Darstellung des Aufbaus einer Anordnung umfassend ein Operationsinstrument 5 und eine erfindungsgemäße Knochenbearbeitungs-Führungsschiene.

Die in Fig. 1-5 dargestellte erfindungsgemäße Knochenbearbeitungs-Führungsschiene definiert eine Verlaufsgerade und umfasst zwei Führungsschienen 1 und 2. Jede dieser Führungsschienen 1 und 2 kann allerdings auch allein als Knochenbearbeitungs-Führungsschiene dienen, z.B. indem ein geeigneter Schlitten für eine Halterung oder indem eine magnetische oder Magnetoberfläche entlang von Laufbahnen vorgesehen ist Diese Führungsschienen 1 und 2 weisen an den zueinander zugewandten Seiten jeweils eine Auswölbung 6 auf, welche sich in Längsrichtung der Führungsschiene erstrecken und je eine Laufbahn 15 ausbilden. Die Auswölbungen 6 der beiden Führungsschienen 1 und 2 bilden einen gemeinsamen Führungskanal zur Aufnahme und Führung eines Operationsinstruments 5 auf. Das Operationsinstrument 5 weist bereichsweise ein kugelförmiges Gehäuse 13 auf, welches als Halterung ausgebildet ist. Unterhalb dieses kugelförmigen Gehäuses 13 ist in Richtung des Knochens 11 eine Öffnung 10 oder ein Ansatz, so dass beispielsweise ein Laserstrahl in Richtung des Knochens 11 abgegeben werden kann. Das Operationsinstrument 5 ist beweglich entlang der Verlaufsstrecke der Knochenbearbeitungs-Führungsschiene angeordnet, allerdings kann das Operationsinstrument in keiner anderen Richtung als entlang dieser Verlaufsgeraden bewegt werden. Ein Kabel und eine entsprechende Kabelführung 4 können oberhalb des kugelförmigen Gehäuses bzw. in entgegengesetzter Richtung zum stiftartigen Ansatz 10 aus dem kugelförmigen Gehäuse des Operationsinstruments 5 hervorstehen. Die Führungsschienen 1 und 2 liegen mit flanschförmigen Kontaktflächen 14 auf dem Knochen 11 auf.

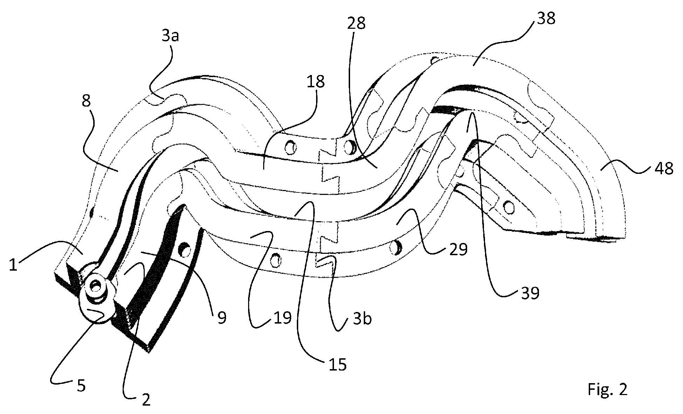

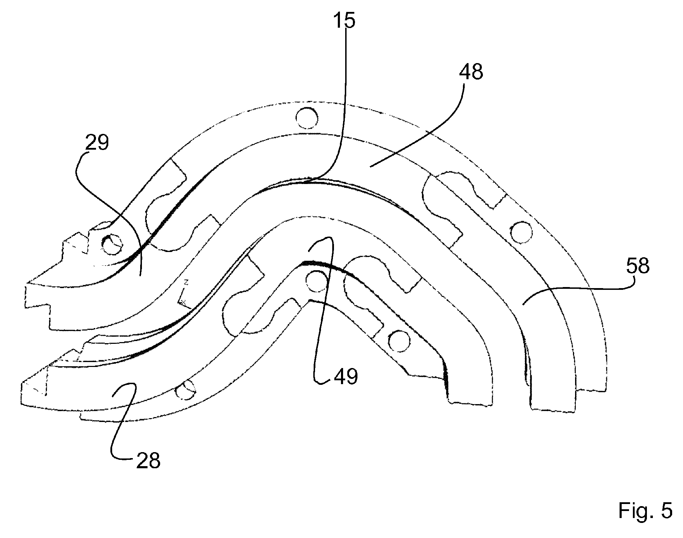

Die Führungsschienen 1 und 2 weisen in einer bevorzugten Ausführungsvariante in Fig. 2 und 5 zumindest zwei Schienenabschnitte 8, 9 und besonders bevorzugt mehr als zwei Schienenabschnitte 8, 9, 18, 19, 28, 29, 38, 39, 48, 49, 58 auf.

In Fig. 1, 3 und 4 ist die Unterteilung in Schienenabschnitte zwar ebenfalls vorhanden jedoch nicht dargestellt.

In Fig. 2 und Fig. 5 sind zudem zwei ineinandergreifende Steckverbindungselemente 3a dargestellt, welches die Schienenabschnitte 8, 9, 18, 19, 28, 29, 38, 39, 48, 49, 58 miteinander verbinden. Die Steckverbindungselemente können im Wesentlichen senkrecht zur Kontaktfläche 14 der Führungsschienen 1 und 2 aneinander herangeführt und miteinander in Formschluss gebracht werden. Dieses besondere Konzept der Steckverbindung ist besonders vorteilhaft gegenüber anderen Verbindungsarten und kann auch auf andere Knochenbearbeitungs-Führungsschienen übertragen werden.

Die Schienenabschnitte 8, 9, 18, 19, 28 können durch verschiedene Steckverbindungen, z.B. durch abgerundete Steckverbindungen 3a oder durch Schwalbenschwanzsteckverbindungen 3b und/oder durch andere Verbindungselemente anderweitig, also nicht durch stecken sondern z.B. durch Verrasten oder Verschrauben miteinander verbunden sein. Die Steckverbindungselemente sind

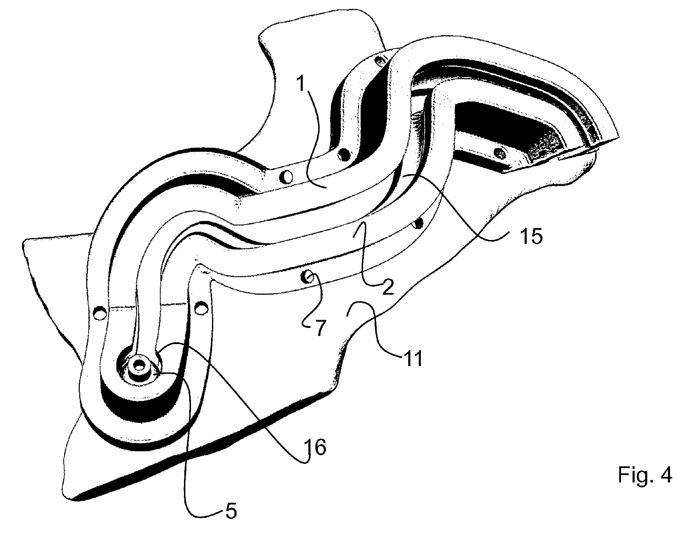

In Fig. 1 und Fig. 2 weisen die Führungsschienen 1 und 2 Löcher 7 auf, welche zum Durchführen von nicht-dargestellten Befestigungsmitteln, z.B. Knochenschrauben vorgesehen sind, um die Führungsschienen an einem Knochen zu verankern. Dies ist in Fig. 4 dargestellt.

Die Führungsschienen 1 und 2 können aus einem oder mehreren Materialien gefertigt sein, so ist es beispielsweise möglich, dass die Schiene im Bereich der Auswölbung ein Kernelement aus einem nicht-kompressiblem Material wie Metall, z.B. Edelstahl, Kunststoff oder Keramik, z.B. Magnesium aufweist und dass die Außenseiten der Schienen zumindest bereichsweise mit einem kompressiblen Material z.B. einem Schwamm oder Schaum bedeckt sind, um Reibungen mit Hautabschnitten, Knochen oder Organen zu vermeiden.

Die Knochenbearbeitungs-Führungsschiene dient der Applikation auf einem Knochen, welcher in Fig. 1 als platter Knochen ausgebildet ist. Die Führungsschienen 1 und 2 können dabei durch verschiedene Verbindungsvarianten mit dem Knochen verbunden werden. Sie können beispielsweise verklebt, verschraubt oder vernietet werden. Sofern ein Klebstoff angewandt wird, kann der Klebstoffzusammensetzung ein Wirkstoffe, z.B. ein antibiotischer und/oder entzündungshemmender Wirkstoff, zugegeben werden, welcher nach dem Ablösen der Knochenbearbeitungs-Führungsschiene gemeinsam mit dem Klebstoff auf der Knochenoberfläche verbleiben und die Heilung unterstützt.

Die Knochenbearbeitungs-Führungsschiene kann auch vollständig aus biologisch abbaubaren, insbesondere aus resorbierbarem, Material bestehen.

Die Schienenabschnitte 8, 9, 18, 19, 28 der Führungsschiene weisen eine mehr oder weniger gerade Form auf. Sie können in eine bevorzugten Ausführungsvariante kundenspezifisch und angepasst auf den Knochen gefertigt werden.

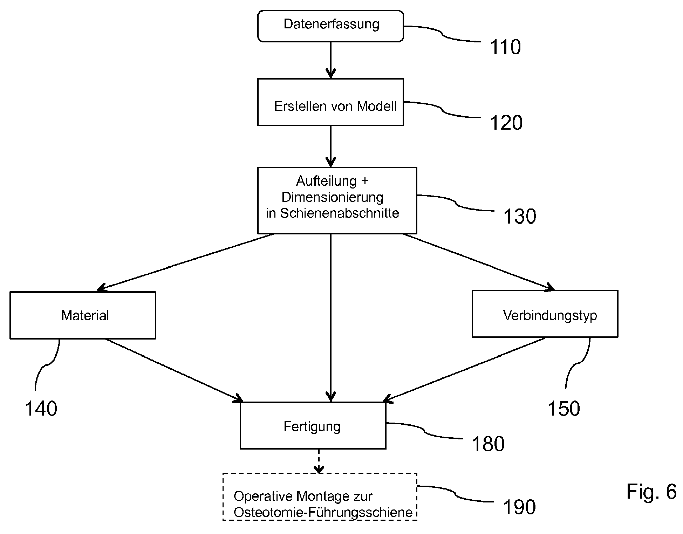

Dabei ist zu beachten, dass für eine optimale Auflage auf dem Knochen und um eine Durchtrennung eines Knochens entlang einer individuell optimierten Verlaufsstrecke die jeweilige Führungsschiene und jede der dazugehörigen Schienenabschnitte individuell gefertigt werden müssen. Ein entsprechendes Verfahren wird ebenfalls im Rahmen der vorliegenden Erfindung erfasst und nachfolgend beschrieben, welches in Fig. 6 näher dargestellt ist.

In einem ersten Schritt erfolgt eine Datenerfassung 110. Bei dieser Datenerfassung handelt es sich um eine individuelle Datenerfassung eines Patienten. Es handelt sich somit um individuelle Patientendaten, welche vorzugsweise auf Basis von Bildgebungsverfahren bzw. bildgebende Verfahren erstellt wurden.

Derlei Verfahren ermöglichen die Aufnahmen von Organen und/oder Knochen auf deren Basis anschließend ein Modell erstellt werden kann. Bildgebende Verfahren basieren bevorzugt auf folgenden Signalen aus welchen im Anschluss ein Bild einzelner Organe, Knochen oder ähnliches für den individuellen Patienten erstellt werden können:
- Kernspinresonanz, z. B. MRT-Aufnahmen
- Ultraschall, z. B. sonographische Aufnahmen
- Röntgenstrahlungen, z. B. Röntgenaufnahmen,
- Infrarotstrahlung z. B. thermographische Aufnahmen,
- Licht im VIS-Bereich, z. B. Endoskopische Aufnahmen
- Impedanz, z. B. EIT-Aufnahmen
- Radionuklide z. B. szintographische Aufnahmen

Besonders bevorzugt sind dreidimensionale Aufnahmen von bildgebenden Verfahren und somit sind bildgebende Verfahren zur Datenerfassung bevorzugt, welche dreidimensionale Aufnahmen ermöglichen.

Zusätzlich zur Erfassung von dreidimensionalen Aufnahmen, kann auch ein Erfassen weiterer chemischer und/oder physikalischer Daten erfolgen. Dies kann beispielsweise das Erfassen der Konzentration spezifischer Verbindungen im Körper, z.B. einer Ionenkonzentration und/oder des pH-Werts, der Körpertemperatur, des Herzschlags und/oder des Kontraktions- und Expansionsvolumens einzelner Organe umfassen. Diese Werte sind teilweise ebenfalls patientenspezifisch.

Auf Basis dieser Datenerfassung kann ein Modell 120 eines betroffenen Knochens und/oder der Umgebung des Knochens erstellt werden. Handelt es sich bei dem Modell um die Umgebung, so kann aufgrund dieses Modells ein Raumbedarf der benötigten Knochenbearbeitungs-Führungsschiene ermittelt werden.

Bei dem Modell 120 kann es sich um ein digitales dreidimensionales ComputerModell handeln oder um ein reales dreidimensionales Computermodell, welches vorzugsweise durch Stereolithographie, besonders bevorzugt durch Rapid-Prototyping, und insbesondere durch ein generatives Fertigungsverfahren, wie z.B. selektives Laserschmelzen oder durch einen 3D-Druck hergestellt wurde.

Anhand dieses Modells 120 werden vor der Ausgestaltung der Knochenbearbeitungs-Führungsschiene Fertigungsvorgaben erstellt, dies umfasst u. a. eine Aufteilung und Dimensionierung 130 von Schienenabschnitten 8, 9, 18, 19, 28 des dreidimensionalen individualisierten Knochenbearbeitungs-Führungsschiene.

Das Modell 120 der Knochenbearbeitungs-Führungsschiene und die herzustellenden Schienenabschnitte können zur Definition ihrer dreidimensionalen Ausbreitung jeweils durch ein imaginäres quaderförmiges Kantenmodell begrenzt werden, wobei diese Kantenmodelle die maximale Ausdehnung des Modells 120 und die maximale Ausdehnung der Schienenabschnitte des Modells aufweisen. Somit ist jeder der Schienenabschnitte kleiner als das Gesamtkonstrukt der Knochenbearbeitungs-Führungsschiene.

Dabei ist das Volumen des Kantenmodells, welches das Modell 120 der Knochenbearbeitungs-Führungsschiene begrenzt, größer als das Volumen des Kantenmodells, welches einen Schienenabschnitt des Modells 120 begrenzt.

Vorzugsweise ist das Volumen des Kantenmodells des Modells 120 der Knochenbearbeitungs-Führungsschiene bzw. der Führungsschiene 1 oder 2 zumindest dreimal größer, besonders bevorzugt zumindest 8-fach größer, als das Volumen des Kantenmodells des Schienenabschnitts 8, 9, 18, 19, 28 des Modells 120.

Die Definition gemäß Kantenmodell bezieht sich dabei auf ein Modell der Knochenbearbeitungs-Führungsschiene. Handelt es sich bei dem Modell um ein Modell der Umgebung, so ist anhand des Modells der dreidimensionale Raumbedarf der benötigten Knochenbearbeitungs-Führungsschiene zu erstellen und das Kantenmodell ist auf Grundlage dieses Raumbedarfs zu erstellen.

Durch das Modell 120 wird das Makrodesign der bereitzustellenden Führungsschienen 8, 9, 18, 19, 28 abgestimmt, welche Führungsschienen somit individuell und optimal in allen drei Raumrichtungen auf den Patienten angepasst sind. Dabei ändern sich typischerweise die Abmessungen des Modells und die nach dem Vorgaben des Modells hergestellte Knochenbearbeitungs-Führungsschiene für jeden Patienten in allen drei Raumrichtungen.

Bevorzugt weisen die beiden Schienen je eine Ausnehmung 16 auf, welche ein Einführen des kugelförmigen Gehäuses in den Führungskanal erlaubt. Alternativ kann auch nur eine der beiden Schienen eine solche Ausnehmung aufweisen.

Nach der Aufteilung des Modells in Schienenabschnitte erfolgt eine segmentspezifische Auswahl zur Ausgestaltung hinsichtlich eines jeweiligen Schienenabschnitts.

Diese Auswahl betrifft u. a. die Auswahl des Materials 140 eines jeweiligen Schienenabschnitts und die Auswahl der Verbindung 150 der Schienenabschnitte untereinander.

Bei der Materialauswahl des Basismaterials können sowohl Metalle als auch keramische Materialien und/oder Kunststoffe genutzt werden. In einer bevorzugten Ausführungsvariante der Erfindung können insbesondere faserverstärkte Kunststoffe für die Herstellung der Schienenabschnitte genutzt werden. Es sind auch Materialkombinationen und Materialverbundstoffe möglich, welche in einem Schienenabschnitt vorkommen können. Dies kann durch die Materialauswahl vorab bestimmt und angepasst sein. Jeder Patient hat dabei andere Körpermaße und muskuläre Ausprägungen, sodass die Dimensionierung der Knochenbearbeitungs-Führungsschiene anhand des vorher erstellten Modells individuell vorgenommen werden kann.

Besonders bevorzugt verwendete Metalle sind Stahl und/oder Titan.

Zu den bevorzugten keramischen Werkstoffen zählen insbesondere auch Werkstoffe aus Magnesium.

Besonders bevorzugt verwendete Kunststoffe sind resorbierbare Kunststoffe vorzugsweise basierend auf Polymilchsäure und/oder deren Derivate, z.B. PLA. Die Kunststoffe können bevorzugt zumindest bereichsweise faserverstärkt sein.

Gleiches gilt für die Auswahl des Typs der Verbindungen zwischen den einzelnen Schienenabschnitten. Die Verbindung kann teilweise flexibel sein. Dies kann über Kugelgelenke erreicht werden, wie sie z.B. in der US 6 060 641 A offenbart sind. Sie kann allerdings auch starr sein. Die Verbindung zwischen dien Schienenabschnitten kann auf vielfältige Weise realisiert werden.

Optional kann auch eine Auswahl hinsichtlich von Sensoren 160 erfolgen, welche in dem Schienenabschnitt integriert sein können. Sensoren können beispielsweise die Hitzeentwicklung am der Nahtstelle beim Durchtrennen des Knochens ermitteln und die Leistung eines Lasers des Operationsinstruments entsprechend einstellen. Entsprechende Sensoren können in mikromechanischer Bauweise als MEMS-Elemente ausgebildet sein.

Weiterhin kann die auch eine Stromversorgungsquelle aufweisen zur Energieversorgung der vorgenannten Aktuatoren und/oder Sensoren.

Weiterhin kann ein Schienenabschnitt eine Auswerteeinheit aufweisen, welche die von einem oder mehreren Sensoren ermittelten Daten erfasst und ggf. auswertet oder an eine externe Recheneinheit, also eine Recheneinheit außerhalb des Körpers, zur weiteren Auswertung, vorzugsweise durch Wireless-Datenübertragung, übermittelt. Zudem kann die Steuereinheit aufgrund der ermittelten Daten einen oder mehrere der vorgenannten Aktuatoren ansteuern. In einer bevorzugten Variante kann die Steuer- und die Auswerteeinheit als ein Bauteil realisiert sein.

Weiterhin optional kann eine Auswahl von Wirkstoff- und/oder Releasesystemen 170 erfolgen, mit welchen ein Schienenabschnitt beschichtet wird oder welche im Material des Schienenabschnitts integriert bzw. eingebunden werden.

Unter dem Begriff Wirkstoffe bzw. Wirkstoffsysteme sind Arzneistoffe zu verstehen. Dies können z.B. Antibiotika, Immunsuppressiva, Wachstumshormone oder Zytostatika sein.

Releasesysteme umfassen alle Substanzen, welche typischerweise zusätzlich dem Arzneistoff zugegeben werden, beispielsweise um die Freisetzung des Arzneistoffes im Körper hinsichtlich des Abgabezeitraumes und des Abgabeortes zu steuern oder um den Arzneistoff unter verschiedenen Bedingungen haltbar zu machen. Typische Substanzen sind z.B. Tocopherol, welches oft als Antioxidans zugesetzt wird, oder Polysaccharide, z.B. Chitin, zur Mikroverkapselung eines entsprechenden Arzneistoffes. Die Wahl des Releasesystems hängt u. a. auch von der Wahl des Materials, also des Basismaterials, ab aus welchem der Schienenabschnitt hergestellt werden soll.

In einem weiteren Schritt erfolgt die Fertigung 180 der Schienenabschnitte entsprechend der vorgenannten Auswahlkriterien. Die jeweiligen Schienenabschnitte können vorzugsweise durch generative Fertigungsverfahren, z. B. SLM-Verfahren, durch 3D-Druckverfahren und/oder durch mechanische, insbesondere materialabtragende Verfahren, wie z. B. Fräsen, anhand des Modells ausgebildet werden.

Die Schienenabschnitte können durch eine operative Montage 190 zu einer Knochenbearbeitungs-Führungsschiene zusammengesetzt werden. Dabei können die Schienenabschnitte im Rahmen eines operativen Eingriffs durch eine Operationswunde in den menschlichen Körper eingeführt und im Körper zur entsprechenden Führungsschiene zusammengesetzt bzw. montiert werden. Die Montage kann zudem die Fixierung der Knochenbearbeitungs-Führungsschiene an einem bestehenden Knochen umfassen.

Das Einführen der Schienenabschnitte ermöglicht eine Verringerung der Dimensionierung einer Operationswunde, sodass das Einsetzen des individuell auf den Patient angepassten Knochenbearbeitungs-Führungsschiene im Rahmen einer minimalinvasiven Vorgehensweise bei einer Operation erfolgen kann, wodurch die Chancen und der Zeitraum einer erfolgreichen Verheilung und der Regeneration deutlich erhöht wird.

Der operative Eingriff umfassend das Einführen der Schienenabschnitte durch die Operationswunde und deren Zusammensetzung zu einem Implantat oder einer Knochenprothese kann endoskopisch, von Hand, navigiert und/oder roboterunterstützt erfolgen.

In einer bevorzugten Ausführungsvariante der Erfindung kann ein Schienenabschnitt oder eine Verbindung zwischen zwei Schienenabschnitten eine Sollbruchstelle aufweisen, in welchem die Knochenbearbeitungs-Führungsschiene bei stärkerer mechanischer Belastung bevorzugt bricht.

Die vorgenannten Ausführungsbeispiele beziehen sich auf den menschlichen Körper. Es versteht sich allerdings bereits aus dem Kontext der vorliegenden Erfindung, dass die Erfindung auch bei Tieren eingesetzt werden kann.

Nachfolgend wird die Funktionsweise der erfindungsgemäßen Führungsschiene und der erfindungsgemäßen Anordnung näher beschrieben.

Durch die Kugelform des Gehäuses des Operationsinstruments 5 und durch die Auswölbungen 6 bzw. den entsprechend-gebildeten gemeinsamen Führungskanal der Führungsschienen 1 erfolgt. Eine Führung des Operationsinstruments entlang einer vorbestimmten Verlaufsstrecke. Die Kabel des Operationsinstruments können durch einen Kabelkanal zwischen den beiden Führungsschienen 1 geführt sein.

Man erkennt, dass durch die erfindungsgemäße Führungsschiene und die Anordnung zweier solcher Führungsschienen eine gezielte Führung eines Operationsinstruments im Körper eines Patienten möglich ist. Außerdem kann eine komplizierte Verlaufsstrecke, z.B. eine gerade oder gekrümmte oder unregelmäßige Schnittstrecke entlang eines Operationsabschnitts durch eine kundenindividuellausgestaltete Führungsschiene vorgegeben werden.

Die Führungsschiene kann mehrteilig ausgebildet sein, so dass sie minimalinvasiv einsetzbar und im Patienten zusammensetzbar und fixierbar ist. Dabei können kompliziertere Schienenabschnitte, welche z.B. höheren mechanischen Belastungen durch das Operationsinstrument unterliegen mit geringerer Größe dimensioniert sein als einfachere z.B. gerade Schienenabschnitte.

Die erfindungsgemäße Anordnung muss nicht zwingend zwei Führungsschienen umfassen, so ist es auch möglich lediglich eine Führungsschiene vorzusehen, auf welcher ein sogenannter Schlitten mit dem Operationsinstrument gleitend oder rollend geführt ist.

Alternativ oder zusätzlich kann auch nur eine Führungsschiene vorgesehen sein, welche eine magnetische Laufbahn aufweist an welcher das Operationsinstrument, insbesondere das kugelförmige Gehäuse, geführt und gehalten wird.

Die vorgenannten Varianten können alternativ zu der Ausführungsvariante der Fig. 1-5 ausgebildet sein oder die in den Figuren dargestellte Ausführungsform kann um die entsprechenden Merkmale noch zusätzlich ergänzt werden.

Das Operationsinstrument ist nicht auf einen Laser beschränkt, sondern kann auch jegliche andere Form von knochenbearbeitendem Instrument sein, so z.B. eine Bohrmaschine, Schleif-, Fräs- oder eine Sägevorrichtung.

Alternativ ist das kugelförmige Gehäuse nicht dem Operationsinstrument 5 zuzuordnen, sondern es ist Teil einer Führungsschiene oder einer Anordnung aus Führungsschienen und dient der Halterung eines Operationsinstruments.

Diesbezüglich sind zahlreiche weitere konstruktive Ausgestaltungsvarianten im Rahmen der vorliegenden Erfindung denkbar.

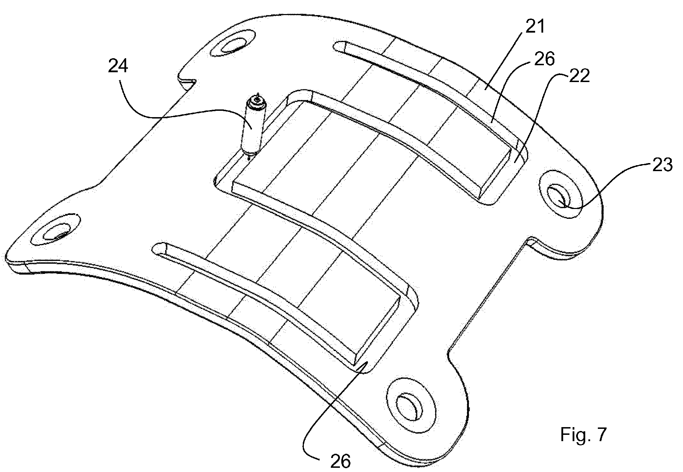

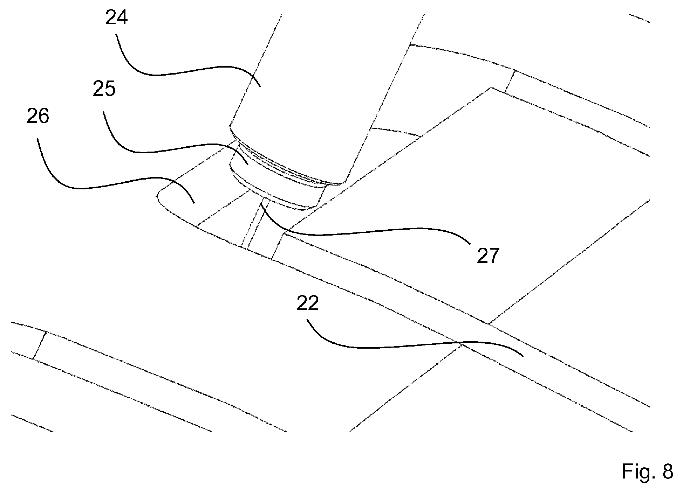

Fig. 7 und 8 zeigen eine zweite Ausführungsvariante einer erfindungsgemäßen Knochenbearbeitungs-Führungsschiene 21 und einer erfindungsgemäßen Anordnung.

Man erkennt eine Knochenbearbeitungs-Führungsschiene welche nicht als Schienenstrang ausgebildet ist, sondern welche eine Plattenform aufweist. In der Knochenbearbeitungs-Führungsschiene 21 ist ein Spalt 22 angeordnet. Der Spalt 22 definiert die vorbestimmte Verlaufsstrecke. Der Spalt 22 weist innenseitig zwei sich gegenüberliegende Laufbahnen 26 auf.

Auf diesen Laufbahnen ist ein Operationsinstrument 24 an einer Halterung 25 geführt. Die Halterung 25 ist im Fall des in Fig. 7 und 8 dargestellten Ausführungsbeispiels ein Wälzlager mit zylindrischer Form. Das Wälzlager kann auf einfache Weise als Kugellager ausgeführt sein und es ist Teil des Operationsinstruments 24. Das Operationsinstrument ist in Fig. 7 und 8 als ein Laser ausgebildet. Ein Laserstrahl 27 ist in Fig. 8 schematisch dargestellt.

Die Knochenbearbeitungs-Führungsschiene 21 verfügt über vier randseitige Löcher 23 durch welche Befestigungsmittel zur Befestigung an einem Knochen oder einer Knochenplatte geführt werden können.

Die Knochenbearbeitungs-Führungsschiene 21 ist in Fig. 7 und 8 nur einteilig dargestellt. Die dargestellte Knochenbearbeitungs-Führungsschiene 21 kann allerdings besonders bevorzugt in mehrere Schienenabschnitte unterteilt sein, um so im Rahmen einer minimalinversiven Operationsstrategie Verwendung zu finden.

### Bezugszeichen

- 1: Führungsschiene
- 2: Führungsschiene
- 3a: abgerundete Steckverbindung
- 3b: Schwalbenschwanzsteckverbindung
- 4: Kabelführung
- 5: Operationsinstrument
- 6: Auswölbung
- 7: Loch
- 8: Schienenabschnitt
- 9: Schienenabschnitt
- 10: Öffnung
- 11: Knochen
- 13: kugelförmiges Gehäuse
- 14: Kontaktfläche
- 15: Laufbahn
- 16: Ausnehmung

- 18: Schienenabschnitt
- 19: Schienenabschnitt

- 28: Schienenabschnitt
- 29: Schienenabschnitt
- 38: Schienenabschnitt
- 39: Schienenabschnitt
- 48: Schienenabschnitt
- 49: Schienenabschnitt
- 58: Schienenabschnitt

- 21: Knochenbearbeitungs-Führungsschiene
- 22: Spalt
- 23: Löcher
- 24: Operationsinstrument
- 25: Halterung
- 26: Laufbahn
- 27: Laserstrahl
- 110: Datenerfassung
- 120: Erstellen von Modell
- 130: Dimensionierung der Schienenabschnitte
- 140: Materialauswahl
- 150: Auswahl der Verbindungstypen
- 180: Fertigung der Schienenabschnitte
- 190: Operative Montage der Knochenbearbeitungs-Führungsschiene

## Patentansprüche

1. Knochenbearbeitungs-Führungsschiene (1, 2, 21) zur Halterung eines Operationsinstruments (5, 24) an einem Knochen (11) und/oder einer Osteosynthese, **dadurch gekennzeichnet, dass** die Knochenbearbeitungs-Führungsschiene (1, 2, 21) eine Laufbahn (15, 26) zur Führung des Operationsinstruments (5, 24) entlang einer vorbestimmten Verlaufsstrecke aufweist.

2. Knochenbearbeitungs-Führungsschiene nach Anspruch 1, **dadurch gekennzeichnet, dass** die Knochenbearbeitungs-Führungsschiene (1, 2) aus zumindest zwei oder mehr Schienenabschnitten (8, 9, 18, 19, 28, 29, 38, 39, 48, 49, 58) gebildet ist, welche gemeinsam die Laufbahn (15) ausbilden.

3. Knochenbearbeitungs-Führungsschiene nach Anspruch 1, **dadurch gekennzeichnet, dass** die Knochenbearbeitungs-Führungsschiene (1, 2) zumindest einen ersten Schienenabschnitt (8, 18, 28, 38, 48, 58) und einen zweiten Schienenabschnitt (9, 19, 29, 39, 49) aufweist wobei der erste Schienenabschnitt (8, 18, 28, 38, 48, 58) einen ersten Laufbahnabschnitt und der zweite Schienenabschnitt(8, 18, 28, 38, 48, 58) einen zweiten Laufbahnabschnitt aufweist, wobei der ersten Laufbahnabschnitt eine stärkere Biegung aufweist als der zweite Laufbahnabschnitt.

4. Knochenbearbeitungs-Führungsschiene nach Anspruch 1 oder 2, **dadurch gekennzeichnet, dass** die Knochenbearbeitungs-Führungsschiene (1, 2, 21), insbesondere jeder der Schienenabschnitte (8, 9, 18, 19, 28, 29, 38, 39, 48, 49, 58) eine Kontaktfläche (14) zur Auflage auf einem Knochen (11) aufweist.

5. Knochenbearbeitungs-Führungsschiene nach einem der vorhergehenden Ansprüche, **dadurch gekennzeichnet, dass** zumindest mehr als 50% aller Schienenabschnitte (8, 9, 18, 19, 28, 29, 38, 39, 48, 49, 58) unterschiedlich zueinander dimensioniert gefertigt sind.

6. Knochenbearbeitungs-Führungsschiene nach einem der vorhergehenden Ansprüche, **dadurch gekennzeichnet, dass** die Schienenabschnitte (8, 9, 18, 19, 28, 29, 38, 39, 48, 49, 58) im zusammengesetzten Zustand einer Knochenbearbeitungs-Führungsschiene (1, 2) ein Kantenmodell mit einem größeren Volumen definieren als jeder einzelne Schienenabschnitt (8, 9, 18, 19, 28, 29, 38, 39, 48, 49, 58), vorzugsweise ein dreifach größeres Kantenmodell, insbesondere ein 8-fach größeres Kantenmodell.

7. Knochenbearbeitungs-Führungsschiene nach einem der vorhergehenden Ansprüche **dadurch gekennzeichnet, dass** die Knochenbearbeitungs-Führungsschiene (1, 2, 21) Anschlussstellen, vorzugsweise für Steckverbindungen, zur Fixierung einer Osteosynthese und/oder eines Implantats und/oder von Verbindungselementen zur Festlegung einer Osteosynthese und/oder eines Implantats zumindest an Teilen der Knochenbearbeitungs-Führungsschiene (1, 2, 21) aufweist.

8. Anordnung umfassend zumindest eine erste Knochenbearbeitungs-Führungsschiene, insbesondere eine Knochenbearbeitungs-Führungsschiene (1, 2, 21) gemäß einer der vorhergehenden Ansprüche, und eine Halterung (13) für ein Operationsinstrument (5) zur Bearbeitung eines Knochens (11) und/oder ein Operationsinstrument (24) zur Bearbeitung eines Knochens (11) mit einer Halterung (25), wobei die Halterung (13, 25) wälz-, roll- und/oder gleitgelagert an der ersten Knochenbearbeitungs-Führungsschiene (1, 21) angeordnet ist.

9. Anordnung nach Anspruch 8, **dadurch gekennzeichnet, dass** die Anordnung zudem eine zweite Knochenbearbeitungs-Führungsschiene, insbesondere eine zweite Knochenbearbeitungs-Führungsschiene (2) nach einem der vorhergehenden Ansprüche, aufweist, wobei jede der beiden Knochenbearbeitungs-Führungsschienen (1, 2) jeweils eine Laufbahn (15) aufweist, wobei beide Laufbahnen (15) Teil eines Führungskanals sind, in welchem das Operationsinstrument (5) mittels der Halterung (13) gleit-, roll- und/oder wälzgelagert angeordnet ist.

10. Anordnung nach einem der vorhergehenden Ansprüche, **dadurch gekennzeichnet, dass** die Halterung (13, 24) eine Kugelform aufweist, welche auf der oder den Laufbahnen (15) aufliegt oder ein zylindrisches Wälzlager aufweist, welches auf den Laufbahnen (26) aufliegt.

11. Anordnung nach einem der vorhergehenden Ansprüche, **dadurch gekennzeichnet, dass** die Anordnung einen Kabelführungskanal aufweist, zur Abdeckung eines Kabels des Operationsinstruments (5, 24).

12. Verfahren zum Bereitstellen von Schienenabschnitten (8, 9, 18, 19, 28, 29, 38, 39, 48, 49, 58) einer mehrteiligen Knochenbearbeitungs-Führungsschiene, insbesondere einer Knochenbearbeitungs-Führungsschiene (1, 2) nach einem der vorhergehenden Ansprüche, vor deren Einführung in einen menschlichen und/oder tierischen Körper,
**gekennzeichnet durch die folgenden Schritte:**
A) Erfassen von Daten (110) eines Patienten, für welchen die Knochenbearbeitungs-Führungsschiene (1, 2) bestimmt ist;
B) Erstellen eines Modells (120) anhand der erfassten Daten;
C) Erstellen von Fertigungsvorgaben für zumindest zwei oder mehr zu einer Knochenbearbeitungs-Führungsschiene (1, 2) zusammensetzbaren Schienenabschnitten (8, 9, 18, 19, 28, 29, 38, 39, 48, 49, 58) auf Grundlage des erstellten Modells wobei die Fertigungsvorgaben umfassen:
C1) eine Dimensionierung der Schienenabschnitte (130) und
D) Fertigung (180) der Schienenabschnitte (8, 9, 18, 19, 28, 29, 38, 39, 48, 49, 58) auf Basis der Fertigungsvorgaben; wobei die Schienenabschnitte (8, 9, 18, 19, 28, 29, 38, 39, 48, 49, 58) miteinander zu einer Knochenbearbeitungs-Führungsschiene (1, 2) montierbar sind.

13. Verfahren nach Anspruch 12, **dadurch gekennzeichnet, dass** das Erstellen von Fertigungsvorgaben für zumindest zwei oder mehr zu einer Knochenbearbeitungs-Führungsschiene (1, 2) zusammensetzbaren Schienenabschnitte (8, 9, 18, 19, 28, 29, 38, 39, 48, 49, 58) auf Grundlage des erstellten Modells wobei die Fertigungsvorgaben umfassen:
C2) eine Materialauswahl (140) bezüglich eines oder mehrerer Materialen für einen jeweiligen Schienenabschnitt (8, 9, 18, 19, 28, 29, 38, 39, 48, 49, 58);
C3) eine Auswahl (150) einer oder mehrerer Verbindungarten (3a, 3b) zwischen den jeweiligen Schienenabschnitten (8, 9, 18, 19, 28, 29, 38, 39, 48, 49, 58);

14. Verfahren nach einem der vorhergehenden Ansprüche, **dadurch gekennzeichnet, dass das** Erstellen des Modells (120) als Erstellen eines dreidimensionalen Computer-Modells, vorzugsweise nach der Finite Elemente Methode, erfolgt.

15. Verfahren nach einem der vorhergehenden Ansprüche, **dadurch gekennzeichnet, dass** das Erfassen von Daten durch ein bildgebendes Verfahren zum Zwecke des Erstellens eines dreidimensionalen Modells erfolgt.
